# EUROPEAN PATENT SPECIFICATION

(11) **EP 1 476 441 B1**
(45) Date of publication and mention of the grant of the patent: **30.05.2007**
(21) Application number: 02806580.3
(22) Date of filing: 01.10.2002
(51) Int. Cl.: C07D 401/12

(54) **A METHOD OF ELIMINATING SULFONE ANALOG IN THE SYNTHESIS OF PYRIDINE-BENZIMIDAZOLE SULFOXIDES**
VERFAHREN ZUR ELIMINIERUNG VON SULFONANALOGA IN DER SYNTHESE VON PYRIDIN-BENZIMIDAZOL-SULFOXIDEN
TECHNIQUE D'ELIMINATION D'ANALOGUE DE SULFONE DANS LA SYNTHESE DE SULFOXYDES PYRIDINE-BENZIMIDAZOLE

(30) Priority: 23.01.2002 TR 200200186
(43) Date of publication of application: 17.11.2004
(73) Proprietor: Ulkar Kimya Sanayii Ve Ticaret A.S., Besiktas 34353 Istanbul (TR)
(72) Inventor: ÜNSAL, Serafettin, Cerkezköy, 59500 Tekirdag (TR)
(74) Representative: Özbay, Cenk
(86) International application number: PCT/TR2002/000058
(87) International publication number: WO 2003/062223

(56) References cited:
- US-B1- 6 245 913

## Description

This invention relates to reducing the amount of sulfone impurity content of the product of formula (I) to below 0.5% which is formed during oxidation reaction of its corresponding sulfide.

Benzimidazole derivatives,2-(2-pridylinethylsulfinyl) benzimidazole derivatives of general formula (I) are produced from their corresponding sulphides by oxidation with an oxidising agent examplified by m-chloroperbenzoic acid, peracetic acid, trifluoroperacetic acid, permaleic acid, sodium bromite, sodiumhypochloride, hydrogen peroxide or any other reagent already described in the bibliography such as those listed by Madesclaire et al., in Tetrahedran,42,5454 (1986).In a number of patents regarding the production of compound (I) various methods are described. For example, in patent WO 00/78729 A1, international publication date 28.12.2000, crystalline forms of Lansoprazole are described. In this patent pharmaceutically advantageous crystalline forms of Lansoprazole are laid open.

In European patent 0302720 A1, date of publication 08.02.1989, production of 2-(2-pyridylmethylsulfinyl)-benzimidazole compounds is described. In this patent, production of compound (I) with the oxidation of its corresponding sulphide with hydrogen peroxide in the presence vanadium compounds in good yield and with low production of by product is laid open.

In another patent WO 98/21202 of publication date 22.05.1998, crystals of benzimidazole derivatives and their production is described. US 6 245 913 describes a process for the preparation of Omeprazole of high purity.

In this patent, a method is described for the production of substantially solvent-free and stable crystals of benzimidazole derivatives. In none of these inventions there is mention to formation or elimination of impurities in the form sulphone as reaction by product when the last phase of oxidation of the corresponding sulphide to sulphoxide is being carried out. Oxidation reactions should be carefully controlled. Over oxidisation and many other factors may cause the formation of sulphonic impurities which are difficult to eliminate by known purification methods such as recrystallisation, due to formation of mixed crystals with sulphoxide. Sulphone derivatives may also form at the earlier stages or even at the beginning of oxidation reaction and may end up with 3-10%by weight as sulphone contaminated finished product.

Chromatographic separation methods are difficult and costly in addition to being not practical for industrial scale productions. Therefore inventors of the present invention had to find a method for eliminating sulphonic derivatives as an impurity mixed with sulphoxide crystals. This is achieved by treating contaminated product with K₂CO₃ in solid form in aqueous alcohol medium at elevated temperatures, filtering the mixture as hot to remove undissolved K₂CO₃ crystals and cooling the solution to precipitate the product. This sulphone-free product is isolated, washed or suspended in water to remove the crystal alcohol and crystal water. Thus obtained crude product is further recrystallised in a suitable solvent.

### DESCRIPTION OF THE INVENTION:

This invention relates to production of sulphone free 2-(2-pyridinylmethylsufinyl)-1H-benzimidazole derivatives of general formula (I) which are of value as a medicine for example as an anti-ulcer agent and so on, 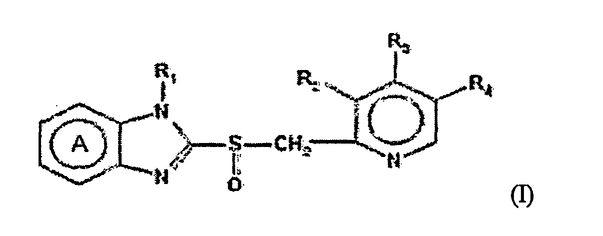 wherein ring A may optionally be substituted, R¹ is an N-protecting group or hydrogen, R²,R³,R⁴ each represents (1) a hydrogen atom, (2) an alkyl group which may be substituted optionally with halogen atom(s) or (3) an alkoxy group which may optionally be substituted with halogen atom(s) or alkoxy; or its salt.

The term "sulphone-free" product used in this text will represent a compound of formula(I) with a sulphone content as an impurity lower than 0.5% as required by many analytical specifications.

Compound (I) is prepared by oxidation of its corresponding sulphide of formula (II) with an oxidizing agent in a suitable solvent. 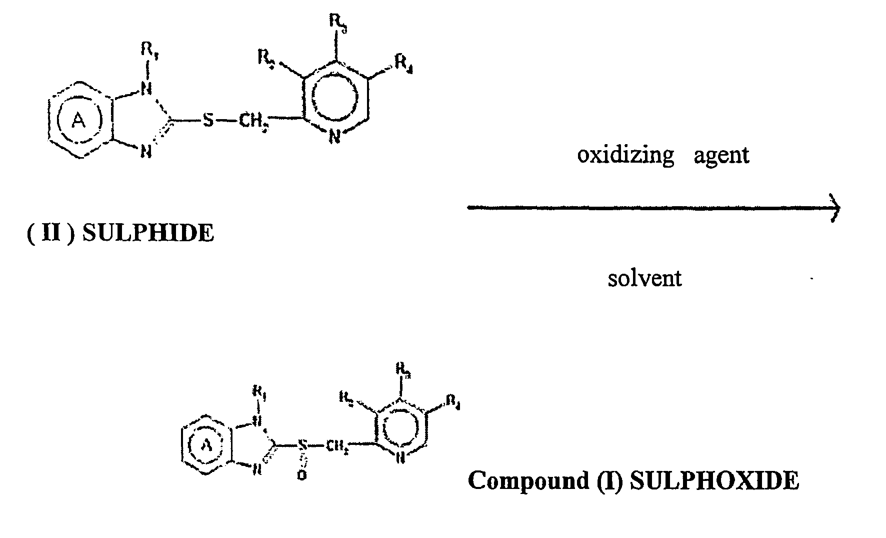 wherein for compound ( II ).R¹,R²,R³,R⁴ has the same meaning as mentioned above for compound (I)

N-protecting group in compound ( I ) and ( II ) is an alkyl group, a carbamoyl group, an alkyl carbamoyl group, an acyl group, a carboalkoxy group, a dialkyl carbomoyl group, an alkylsulfonyl group, an alkoxy carbonyl methyl group or an alkylcarbonylmethyl group,
R¹ in compound (I) and (II) is a hydrogen group,
Ring A of compound (I) and (II) is an alkoxy group, which may optionally be substituted with halogen,
Ring A of compound (I) and (II) is unsubstituted, R³ in compound (I) and (II) is C_{1- 4} alkoxy which may optionally be substituted with fluorine(s) or C_{1- 4} alkoxy -C_{1- 8} alkoxy R⁴ is methyl or hydrogen atom

The salt of formula (I) is pharmaceutically acceptable salt. Example of salts are salts of inorganic bases, salts of organic bases or salts of basic amino acids. Preferred ones are the salts of inorganic bases of alkali metals sodium or potassium,salts of alkaline earth metals such as calcium and magnesium and ammonia salts. Salts of organic bases are preferred as of trimethyl amine, triethyl amine, picoline, ethanol amine, pyridine, diethanol amine, triethanol amine.

Preferred salts of basic amino acids are of lysine, arginine, etc.

Compounds of general formula ( I ) are known as common international denominations
Such as :
Omeprazole[ A ring substituted with OCH₃ , R₂ and R₄= CH₃ and R₃ = OCH₃]
Lansoprazole[A ring is not substituted, R² = CH₃, R³ = OCH₂-CF₃, R⁴ = H]
Pantoprazole[A ring substituted with -OCF₂, R² = CH₃, R³ = OCH₃ and R⁴ = H]

Oxidation reactions can be performed with numerous oxidizing agents such as those listed by Madesclaire et al., in Tetrahedron, 42, 5459 (1986) also by use of m-chloroperbenzoic acid, sodiummetaperiodate, iodobenzene, hydrogen peroxide using Vanadium catalysts .Inventors have experienced that use of m-chloroperbenzoic acid present many advantages as far as formation of low content of sulphonic by-products are concerned. The oxidizing agent is used preferably in approximately equivalent or a little excess amount relative to sulphide compound, more preferably about 1-1.5 equivalent. Also the use of dichloromethane and chloroform as reaction solvent cooled to -20°C or lower reaction temperatures helps low accumulation of impurities.One other factor that helps low formation of sulphonic derivatives is the slow addition of oxidizing agent as within 8-10 hours.

These precautions taken for controlled oxidation will not prevent the over oxidation of sulphoxides to their sulphone analogs.

There are many factors which will accelerate this formation, such as reaction temperature, type of oxidizing agent employed, quality of the compound ( II ), reaction time, type of the solvent in which the reaction is carried out etc. Sulphonic impurity can be isolated and well characterized by means of its spectral data and verified under the HPLC analysis. HPLC is capable of identifying the sulphone analogs along with other impurities and determining their amounts both during the reaction and in the isolated finished product.

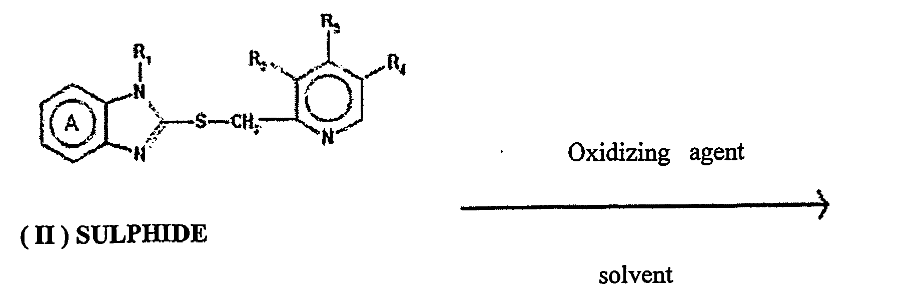

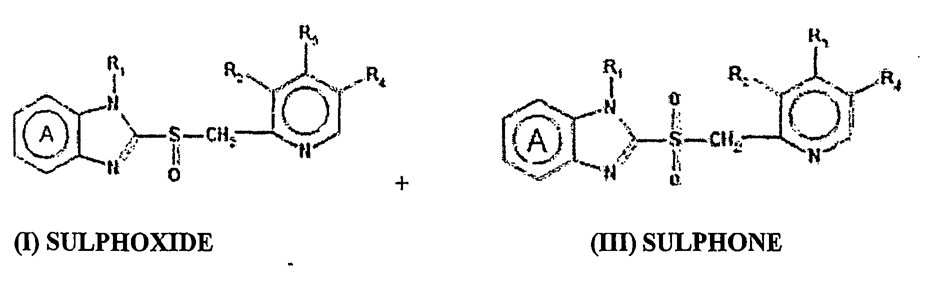

Inventors of the present invention had to develop a process to eliminate this impurity for the purpose of providing sulphone-free crystals of Omeprazole, Lansoprazole and Pantoprazole which are of value as medicine.

### DETAILS OF THE INVENTION:

At the end of oxidation reaction, that is when all of product (II) is converted to its sulphoxide derivative (I), amount of sulphonic impurity formed as by-product is determined by HPLC. Reaction solvent is evaporated and to the precipitate formed aqueous 90-96 % lower alcohol preferably 90% ^{v}/ᵥ ethyl alcohol is added. Slurry is heated to 55-60 °C to obtain a solution. To this solution K₂CO₃ crystals are added and the mixture was kept under stirring at the same temperature for a period of time. Hot mixture is filtered to remove the insolubles mainly consisting of undissolved K₂CO₃ crystals. Clear filtrate is ice- cooled so as to precipitate the solvated and sulphone-free crystals of the product of formula (I). Precipitate is recovered by filtration. Crystals of this compound contain water and alcohol in its crystal structure. Crude product is washed with plenty of water to remove the alcohol and basic water (K₂CO₃ is dissolved in water) or suspended in water as described in patent of international publication number: WO 98/21201. Crude product may optionally be dried or a well-drained wet cake is further subjected to recrystallisation in a suitable solvent.

It is of high importance that residual solvent in the crystal structure of the crude product must be as low as possible in advance of recrystallisation process. Amount of solid K₂CO₃ that should be added to aqueous ethyl alcohol solution of product ( I ) is related to the amount of product (III), that is amount of sulphonic impurity present. HPLC analysis of the oxidation reaction product can easily determine the quantity of sulphone analog on peak area basis. Inventors have experienced that this may be 1 % or higher upto 10 %.

Although there is no formula to calculate the exact amount of added K₂CO₃, over usage than necessary will cause lower yields due to partial decomposition of sulphoxide.

Insufficient usage however, will not reduce the sulphone under desired limits (single impurity limit, not more than 0.5 %) as required by many analytical specifications.

Many experiments were done concerning the optimum amount of solid K₂CO₃ that should be added. Best results were obtained as follows:
- If sulphone content of the contaminated sulphoxide is between 1-5 %; amount of K₂CO₃ that should be added is 10 % by weight of the sulphide charged for oxidation.
- If sulphone content is higher than 5 %; weight of K₂CO₃ added is 15-20 % of weight of the sulphide subjected to oxidation.

These are preferred figures and do not limit the scope of the invention.

The alcohol used as treatment medium includes C₁₋₆ alcohols (e.g. ethanol,isopropyl alcohol,methanol,etc.). Best results were obtained by using ethanol. Aqueous alcohol may be 90% ^{v}/v to 96% ^{v}/v in concentration. Aqueous alcohol particularly preferred is 90% ^{v}/v ethyl alcohol.

Amount of aqueous alcohol in which the sulphoxide dissolved is optional, however excess amounts will cause lower yields due to known solubility property of the product in ethyl alcohol. Preferred amount will be 5 to 10 fold of the weight of the sulphide subjected to oxidation. Solid K₂CO₃ addition and mixing temperature in alcohol medium is 40-65 °C. Preferred treatment temperature is 55-60 °C. Treatment period of alcoholic solution of sulphoxide with K₂CO₃ crystals is about 20-30 minutes. Thus treated mixture is filtered as still hot by the known methods of filtration to eliminate the remaining undissolved K₂CO₃ crystals along with other insolubles. Usage of filtration aids such as celites, hyflosupercell etc, is recommended to achieve a clear filtrate. Filtrate is cooled to about -5 °C and kept under agitation for several hours (2-3 hours) at this temperature so the product is precipitated. Precipitate is either vacuum filtered or centrifuged. Wet cake is washed several times with plenty of deionized water in order to remove alcoholic mother liquor and to remove crystal water or suspended in water at room temperature and stirred for 1-2 hours and re-filtered and washed with water for the same purpose.

Filtered cake is sucked to dryness under vacuum, or the centrifuge is spinned to dryness in order to minimize remaining water or the product may optionally be dried in vacuo at a temperature of 20-60 °C, preferably at 50 °C for about 10-20 hours.

Thus obtained sulphon-free crude product is subjected to recrystallisation in a suitable solvent. For Omeprazole, Lansoprazole and Pantoprozole better crystalline structures possessing the desired whiteness have been obtained by use of ethyl acetate as recrystallisation medium.

Details of this invention are illustrated in the following examples, but invention is not limited to these examples.

### EXAMPLE 1

### Production of 2-[[3-methyl-4-(2,2,2-trifluoroethoxy)-pyridin-2-yl] methyl sulfinyl] benzimidazole :

40 kg of 2-[[[3-methyl-4-(2,2,2-trifluoroethoxy)-pyridin-2-yl]-methyl]thio] benzimidazole monohydrate was added into a vessel containing 1400 kg of chloroform. Mixture was cooled to -20°C with stirring. At this temperature 32 kg of m-chloroperbenzoic acid dissolved in 560 kg of chloroform was added to reaction vessel very slowly. Rate of addition was so adjusted that it took 8 hours to end the oxidation. Amount of the unreacted sulphide and the amount of sulphonic impurity being formed was determined by HPLC at the end of reaction.

Unreacted sulphide was 0.8 % and sulphone analog formed was 3.3 % calculated as based on peak area percentage. To the reaction medium 500kg water was added and temperature was allowed to rise to 20-25°C. At this temperature 120 kg of 25 % ^{w}/_{w} K₂CO₃ solution was added and pH of mixture was measured as 9.9. Phases were separated. Organic phase was distilled off under reduced pressure to dryness. To the remaining off white residue 400 liters of 90 % ^{v}/ᵥ ethanol was added and the mixture was heated at 60°C. At this temperature to the clear solution 4 kg of solid K₂CO₃ was added, and keeping the temperature same, mixture was stirred for 30 minutes. Some of the K₂CO₃ crystals remained undissolved. The mixture as hot was filtered to crystallization vessel and gradually cooled to -5°C. Precipitated product was left under agitation for 3 hours at this temperature fallowed by centrifugation. Centrifuge was spinned to dryness and washed with 1500 liters of deionized water three times. Centrifuge was spin dried. Wet cake was directly taken to recrystallisation. A sample of crude product was analyzed by HPLC. Results were found in the crude product as sulphide content 0.1 % and sulphone analog 0.3 %.

### Recrystallisation of Crude Product:

40 kg of wet crude product as obtained above was added into 800 liters of ethyl acetate and vessel content was heated to 55°C. To the clear solution obtained active carbon was added, and mixture was filtered. Clear and colorless solution was heated and ethyl acetate was distilled off under reduced pressure till about 200 liters of solution remained in the vessel. Vessel content was cooled to -5°C with stirring and the slurry was aged for 3 hours. Pure product was centrifuged and dried for 8 hours at 40°C in vacuo to yield 24 kg of as white powder.

### EXAMPLE 2

### Production of 2-[[3-methyl-4-(2,2,2-trifluoroethoxy)-pyridin-2-yl] methyl sulfinyl] benzimidazole :

To a mixture of 2-[[[3-methyl-4-(2,2,2-trifluoroethoxy)-pyridin-2-yl]-methyl] thio] benzimidazole monohydrate (20g) in chloroform (500ml) was added dropwise peracetic acid (14g) mixed in chloroform (200ml) at -20°C over 8 hours. At the end of reaction, sulphone impurity mixed in crude product was determined as 3.7 % by HPLC analysis. 250 ml water was added to the reaction mixture and heated to 20-25°C. 60 g of 25 % K₂CO₃ solution was added and pH of the mixture was measured as 8.7. Phases were let to separate. Organic phase was distilled to dryness under vacuum. To the remaining residue 200 ml of 90 % ^{v}/ᵥ aqueous ethyl alcohol was added and the mixture was heated at 55°C for about 30 minutes. To the clear solution obtained, 3.5 g of K₂CO₃ crystals were added followed by agitation for 30 minutes at the same temperature. Mixture was filtered as hot and was cooled down to -5°C to precipitate the crude product. Precipitate was stirred at - 5°C for 4 hours and then filtered. Filter was sucked to dryness and then washed with 1 liter of deionized water three times. A sample was taken from wet cake for HPLC analysis. Sulphone analog was found to be 0.4 % 24.5 g of wet crude product was obtained. This product was then taken to recrystallisation in ethyl acetate as described in example 1 (recrystallisation of crude product).

## Claims

1. A method for producing pyridine benzimidazole sulfoxide compounds of general formula (I) 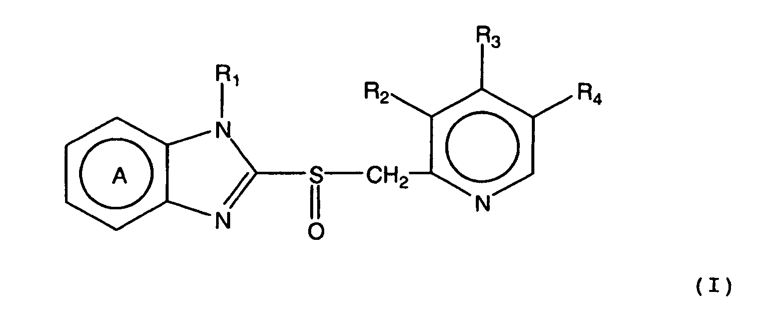 wherein the ring A may optionally be substituted, R¹ is hydrogen or an N-protecting group, each of R², R³, R⁴ represent (1) a hydrogen atom, (2) an alkyl group which may be substituted with halogen atom(s) or (3) an alkoxy group which may be optionally substituted with halogen atom(s) or alkoxy comprising less than 0.5 % of the corresponding sulphone; or their salts, **characterized by** the steps of treating sulphone-contaminated products of compounds of general formula (I) with solid K₂CO₃ in an aqueous alcohol at elevated temperatures and precipitating the compounds of general formula (I).

2. The method of claim 1, **characterized in that** said aqueous alcohol comprises a C₁-C₆ aqueous alcohol.

3. The method of claim 2, **characterized in that** said C₁-C₆ aqueous alcohol of claim 2 is selected from the group comprising ethanol, isopropanol, methanol.

4. The method of anyone of claims 1 to 3, **characterized in that** said aqueous alcohol comprises a 90 to 96 % (v/v) alcohol.

5. The method of claim 4, **characterized in that** said aqueous alcohol comprises a 90 % (v/v) aqueous alcohol.

6. The method of anyone of claims 1 to 5, **characterized in that** said alcohol comprises 90 % (v/v) ethanol.

7. The method of anyone of claims 1 to 6, **characterized in that** treating said sulphone-contaminated products of compounds of general formula (I) is performed at temperatures of about 40 °C to about 60 °C.

8. The method of anyone of claims 1 to 7, **characterized in that** said treating is performed for about 20-30 min.

9. The method of anyone of claims 1 to 8, **characterized in that** said precipitating is performed by cooling said aqueous solution to obtain precipitated products (I), comprising less than 0.5 % of the corresponding sulphone.

10. The method of anyone of claims 1 to 9, **characterized by** the steps of
a) oxidizing sulphide compounds of general formula (II) 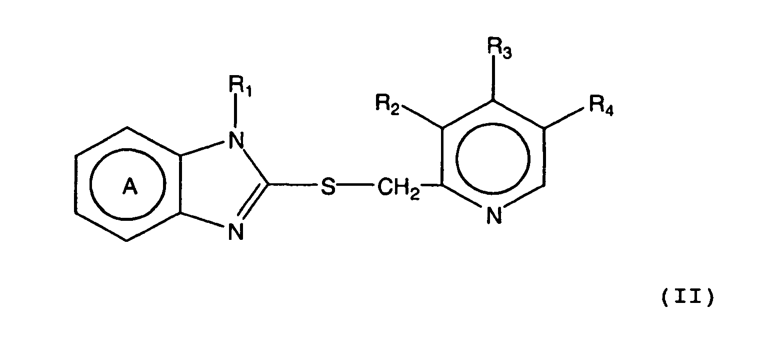 wherein the ring A may optionally be substituted, R¹ is hydrogen or an N-protecting group, each of R², R³, R⁴ represent (1) a hydrogen atom, (2) an alkyl group which may be substituted with halogen atom(s) or (3) an alkoxy group which may be optionally substituted with halogen atom(s) or alkoxy;
in the presence of an oxidizing agent in a solvent;
b) evaporating the solvent to form a precipitate containing sulphone-contaminated products of compounds of general formula (I);
c) treating sulphone-contaminated products of compounds of general formula (I) with solid K₂CO₃ in aqueous alcohol at elevated temperatures to obtain compounds of general formula (I) containing less than 0.5 % of the corresponding sulphone.

11. The method of claim 10, **characterized in that** step c) further comprises the steps of
c1) adding aqueous alcohol to the precipitate of step b);
c2) heating the slurry at temperatures of about 40 °C to about 65 °C to obtain a solution;
c3) adding solid K₂CO₃ to the heated solution;
c4) isolating pyridine benzimidazole sulfoxide compounds of general formula (I), comprising less than 0.5 % of the corresponding sulphone.

12. The method of claim 10 or 11, **characterized in that** the oxidizing agent is selected from the group comprising peracetic acid, m-chloroperbenzoic acid, sodiummetaperiodate, iodobenzene, hydrogen peroxide.

13. The method of anyone of claims 10 to 12, **characterized in that** the amount of oxidizing agent used is approximately equivalent to the amount of compounds of general formula (II) used.

14. The method of anyone of claims 10 to 13, **characterized in that** the amount of solid K₂CO₃ to be added is about 10 % of the weight of the sulphide compound subjected to oxidation if the sulphone content in step c is between 1-5 % by weight.

15. The method of anyone of claims 10 to 13, **characterized in that** the amount of solid K₂CO₃ to be added is about 15 - 20 % of the weight of the sulphide compound subjected to oxidation if the sulphone content in step c is higher than 5 % by weight.

16. The method of anyone of claims 10 to 15, **characterized in that** the amount of aqueous alcohol to be added is about 5 to about 10 fold of the weight of the sulphide compound subjected to oxidation.

17. The method of anyone of claims 11 to 16, **characterized in that** step c4) comprises the steps of
- removing undissolved K₂CO₃ by filtration and/or centrifugation to obtain a solution;
- ice-cooling of the solution to obtain precipitated products, comprising less than 0.5 % of the corresponding sulphone.

18. The method of claim 17, **characterized by**
- isolating the precipitated products, comprising less than 0.5 % of the corresponding sulphone by filtration and/or centrifugation;
- washing the isolated precipitated products with deionized H₂O;
- isolating the washed products by filtration or centrifugation;
- recrystallizing isolated products in ethyl acetate.

19. The method of anyone of claims 1 to 18, **characterized in that** the pyridine benzimidazole compounds are selected from the group comprising omeprazole, lansoprazole and pantoprazole.

## Patentansprüche

1. Eine Methode zur Produktion der pyridenen benzimidazolen sulpoxiden Komponenten nach allgemeinen Formel (I), 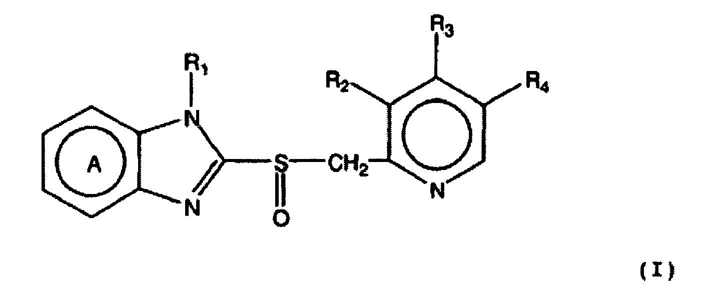 wobei der Ring A optional substituiert werden könnte, R¹ ist ein N-Produkt oder Wasserstoff, jeder einzelne von R²,R³,R⁴ repräsentiert (1) einen WasserstoffAtome, (2) ist eine Alkyl-Gruppe, die optional durch eine halogene Atome(n) oder (3) eine Alkoxy-Gruppe, die optional durch eine halogene Atome(n) oder Alkoxy-Zusammensetzung, die weniger als 0,5 % korrespondierte Sulphone beinhaltet, substituiert werden könnten; oder durch ihren Salz,; **dadurch gekennzeichnet, dass** die Komponente nach Formel (I) durch Sulphone kontaminierte Produkten bei hoher Temperatur in einem wässrigem Alkohol mit soliden K₂CO₃ behandelt wird und die Komponente nach Formel (I) stufenweise bei gleicher Temperatur präzipitiert wird.

2. Methode nach Anspruch 1, **dadurch gekennzeichnet, dass** das schon erwähnte wässrige Alkohol wässrige C₁-C₆ -Alkohole beinhaltet.

3. Methode nach Anspruch 1, **dadurch gekennzeichnet, dass** das im Anspruch 2 benannte wässrige Alkohol Ethanol, Isoproponal, Methanol beinhaltet

4. Methode nach Ansprüche 1 bis 3, **dadurch gekennzeichnet, dass** das schon benannte wässrige Alkohol zwischen von 90 % bis 96 % (^{v}/ᵥ) Alkohol beinhaltet.

5. Methode nach Anspruch 4, **dadurch gekennzeichnet, dass** das schon erwähnte wässrige Alkohol um 90 % (^{v}/ᵥ) wässrige Alkohol beinhaltet.

6. Methode nach Ansprüche 1 bis 5, **dadurch gekennzeichnet, dass** das schon benannte wässrige Alkohol um 90 % (^{v}/ᵥ) Ethanol beinhaltet.

7. Methode nach Ansprüche 1 bis 6, **dadurch gekennzeichnet, dass** die Komponente nach Formel (I) mit dem schon benannten, durch Sulphone kontaminierten Produkten bei der Temperatur 40 C° bis 60 C° behandelt werden.

8. Methode nach Ansprüche 1 bis 7, **dadurch gekennzeichnet, dass** die schon benannte Verfahren zur Behandlung etwa zwischen 20 bis 30 Minuten dauert.

9. Methode nach Ansprüche 1 bis 8, **dadurch gekennzeichnet, dass** die schon benannte Präzipitation durch die Abkühlung der schon erwähnten wässrigen Solution, die weniger als 0,5 % Sulphone beinhaltet, über die präzipitierte Produkte (I) erfolgt.

10. Methode nach Ansprüche 1 bis 9, **gekennzeichnet durch** die Schritte;
a) Oxidation in einer Solution, die in sic oxidierende Stoffe beinhaltet, deren Formel (II) über die Oxidation der Sulphide-Komponenten nachstehend angegeben ist, 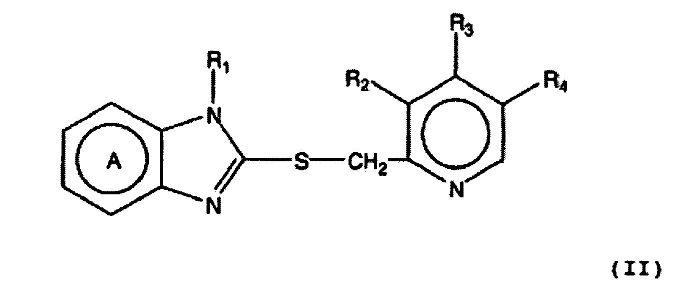 wobei der Ring A optional substituiert werden könnte, R¹ ist Wasserstoff oder ein N-Schutz-Gruppe, jeder einzelne von R^{2,}R³,R⁴ repräsentiert (1) einen Wasserstoff- Atome, (2) ist eine Alkyl-Gruppe, die optional **durch** eine halogene Atome(n) oder (3) eine Alkoxy-Gruppe, die optional **durch** eine halogene Atome(n) oder Alkoxy;
in Anwesenheit eines Oxidationsmittel in einem Solvent;
b) Verdampfung der Komponente, deren allgemeine Formel (I) ist, so dass, eine Solvenz erzeugt werden kann, die mit Sulphon kontaminierte Produkte beinhaltet;
c) Zur Erzeugung der Komponente nach der allgemeinen Formel (I), die wenigstens um 0,5 % Sulphone beinhaltet und mit **durch** Sulphone-Komponente kontaminierten Produkten bei hoher Temperatur mit wässrigem Alkohol, mit soliden K₂CO₃ behandelt wird.

11. Methode nach Anspruch 10, **gekennzeichnet durch** den Schritt c) und zusätzlich sowie Schritte;
c1) Zufügung des wässrigen Alkohols zur im Schritt b) beschriebenen Solution;
c2) Zur Erzeugung einer Solution Erwärmung der Mischung etwa zwischen 40 C° und 60 C°;
c3) Zufügung an die erwärmte Solution von soliden K₂CO₃ Komponenten;
Isolierung der pyridinen benzimidazolen Sulfoxid - Komponenten, welche nach allgemeiner Formel (I) um weniger als 0,5 % entsprechende Sulphone beinhaltet.

12. Methode nach Anspruch 10 oder 11, **dadurch gekennzeichnet, dass** die Oxidationsmittel aus pärasetischen Säuren, m-chloroperbenzoischen Säuren, Natrium-metaperiodaten, Iodobenzenen, wasserstofflichen Peroxyden ausgewählt werden.

13. Methode nach Ansprüche 10 bis 12, **dadurch gekennzeichnet, dass** die Menge der angewandten Oxidationsmittel die gleiche Menge enthält, die nach allgemeinen Formel (II) für Komponente verwendet wird.

14. Methode nach Ansprüche 10 bis 13, **dadurch gekennzeichnet, dass** wenn die Menge der addierten soliden K₂CO₃ im Vergleich zu dem im Schritt c beschriebenen Sulphone-Inhalt ca. um 1 % bis 5 % wägt, das deren Gewicht 10 % der zu oxidierenden Sulphiden-Komponente beträgt.

15. Methode nach Ansprüche 10 bis 13, **dadurch gekennzeichnet, dass** wenn die Menge der addierten soliden K₂CO₃ im Vergleich zu dem im Schritt c beschriebenen Sulphone-Inhalt mehr als 5 % wägt, das deren Gewicht zwischen 15 % und 20 % der zu oxidierenden Sulphiden-Komponente beträgt.

16. Methode nach Ansprüche 10 bis 15, **dadurch gekennzeichnet, dass** die Menge des addierten wässrigen Alkohols ca. 5 bis 10 Mal mehr als die der zu oxidierenden Sulphiden-Komponente beträgt.

17. Methode nach einem der Ansprüche 11 bis 16, **gekennzeichnet durch** Schritt c4) enthaltend folgende Schritte;
- Zur Erzeugung einer Solution Entfernung der undissolvierten K₂CO₃ **durch** Filtration und/oder Zentrifuge;
- Abkühlung der Solution mit Eis, um die präzipitierte Produkte, die weiniger als 0,5 % Sulphone enthalten, zu erzeugen.

18. Methode nach Anspruch 17, **gekennzeichnet durch**
- Isolierung der präzipitierten Produkte, die weiniger als 0,5 % Sulphone enthalten, **durch** Filtration und/oder Zentrifuge.
- Waschen der isolierten präzipitierten Produkte mit Deionisierung **durch** H₂O;
- Isolierung der gewaschenen Produkten **durch** Filtration oder Zentrifuge;
- Rekristallisation der isolierten Produkte im Ethylassetat.

19. Methode nach Ansprüche 1 bis 18, **dadurch gekennzeichnet, dass** pyridinen benzimidazolen Sulfoxid-Komponente aus den Gruppen ausgewählt werden, welche Omeprazole, Lansoprazole und Pantoprazole enthält.

## Revendications

1. Une méthode de produire composés pyridine benzimidazole sulphoxide de formule générale (I) 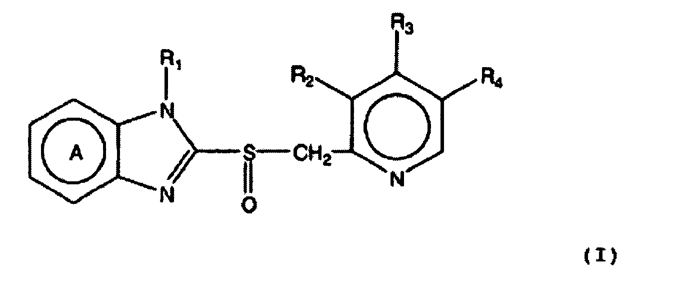 dans laquelle le rond A peut substitué optionnellement, R¹ est l'hydrogène ou un group N-protecteur, chacun de R², R³, R⁴ représente (1) une atome d'hydrogène, (2) un group d'alcali qui peut être substitué par atome(s) d'halogène ou (3) un group d'alkoxy comprenant moins de 0.5 % du sulfone correspondant ; ou leurs sels, **caractérisé par** les pas de traitement des produits sulfone contaminés des composés de la formule générale (I) avec K₂CO₃ solide dans une alcool aqueux aux températures élevées et de précipiter les composés de la formule générale (I).

2. Méthode selon la revendication 1, **caractérisé en ce que** ledit alcool aqueux comprend un alcool aqueux de C₁-C₆.

3. Méthode selon la revendication 2, **caractérisé en ce que** ledit alcool aqueux de C₁-C₆ de la revendication 2 est choisi entre un group comprenant l'éthanol, l'isopropanole, méthanol.

4. Méthode selon l'une quelconques de revendications 1 à 3, **caractérisé en ce que** ledit alcool aqueux comprend l'alcool de 90 à 96 % (^{v}/ᵥ).

5. Méthode selon la revendication 4, **caractérisé en ce que** ledit alcool aqueux comprend l'alcool aqueux de 90 % (^{v}/ᵥ).

6. Méthode selon l'une quelconques de revendications 1 à 5, **caractérisé en ce que** ledit alcool comprend l'éthanol de 90 % (^{v}/ᵥ).

7. Méthode selon l'une quelconques de revendications 1 à 6, **caractérisé en ce que** le traitement desdits produits des composés de sulfone contaminé de la formule générale (I) est perfectionné dans les températures d'environ 40 °C à l'environ 60 °C.

8. Méthode selon l'une quelconques de revendications 1 à 7, **caractérisé en ce que** ledit traitement est perfectionné pendant environ 20-30 minutes.

9. Méthode selon l'une quelconques de revendications 1 à 8, **caractérisé en ce que** ledit précipité est perfectionné en refroidissant ladite solution aqueuse d'obtenir les produits précipités (I), comprenant le taux inférieure de 0.5 % du sulfone correspondant.

10. Méthode selon l'une quelconques de revendications 1 à 9, **caractérisé par** les pas suivant :
a) l'oxydation des composés de sulfite de la générale formule (II) dans lequel 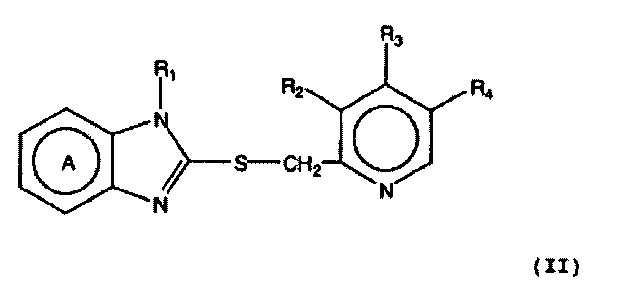 le rond A peut être substitué optionnellement, R¹ est l'hydrogène ou un group N-protecteur, chacun de R², R³, R⁴ représente (1) une atome d'hydrogène, (2) un group d'alcali qui peut être substitué par atome(s) d'halogène ou (3) un group d'alkoxy qui peut être substitué par atome(s) d'halogène ou alkoxy ;
dans la présence d'un agent d'oxydant dans un solvant ;
b) l'évaporation du solvant afin de former un précipité contenant les produits des sulfones contaminés des composés de la formule générale (I) ;
c) le traitement des produits sulfone contaminés des composés de la formule générale (I) avec K₂CO₃ solide dans l'alcool aqueux aux températures élevées d'obtenir les composés de la générale formule (1) contenant le sulfone correspondant moins de 0.5 %.

11. Méthode selon la revendication 10 **caractérisé en ce que** le pas c) comprend aussi les pas ci-dessous :
c1) l'addition l'alcool aqueux au précipité de pas b);
c2) le réchauffement du composé aux températures d'environ 40 °C à l'environ 65 0C d'obtenir une solution ;
c3) l'addition K₂CO₃ solide à la solution réchauffée ;
c4) l'isolation des composés de pyridine benzimidazole sulphoxide qui contiennent le sulfone correspondant moins de 0.5 % de la formule générale (1).

12. Méthode selon l'une des revendications 10 ou 11, **caractérisé en ce que** l'agent d'oxydant est choisi entre un group comprenant peracetic acide, m-chloroperbenzoic acide, sodiummetaperiodate, iodobenzène, peroxyde d'hydrogène.

13. Méthode selon l'une quelconques de revendications 10 à 12, **caractérisé en ce que** la quantité utilisée d'agent d'oxydant est presque équivalent à la quantité utilisée des composés de la formule générale (II).

14. Méthode selon l'une quelconques de revendications 10 à 13 **caractérisé en ce que** la quantité de K₂CO₃ solide qui sera ajoutée est environ 10 % du poids du compose de sulfite assujetti à l'oxydation si le contenu du sulfone dans le pas c) est entre 1-5 % au poids.

15. Méthode selon l'une quelconques de revendications 10 à 13 **caractérisé en ce que** la quantité de K₂CO₃ solide qui on ajoutera est environ 15-20 % du poids du compose de sulfite assujetti à l'oxydation si le contenu du sulfone dans le pas c) est supérieur à 5 % au poids.

16. Méthode selon l'une quelconques de revendications 10 à 15 **caractérisé en ce que** la quantité de l'alcool aqueux qui on ajoutera est multiple d'environ 5 à l'environ 10 du poids du composé sulfite assujetti à l'oxydation.

17. Méthode selon l'une quelconques de revendications 11 à 16 **caractérisé en ce que** le pas c4) comprend les pas ci-dessous :
- la suppression de non dissous K₂CO₃ par filtration et/ou par centrifugation d'obtenir une solution ;
- le refroidissement de la solution d'obtenir les produits précipités comprenant le sulfone correspondant moins de 0.5 %.

18. Méthode selon la revendication 17 **caractérisé par**
- isolation des produits précipités comprenant le sulfone correspondant moins de 0.5 % par filtration et/ou par centrifugation ;
- lavage des produits précipités à H₂O déionisé ;
- isolation des produits lavés par filtration et/ou par centrifugation
- recristallisation des produits lavés dans éthyl acétate.

19. Méthode selon l'une quelconques de revendications 1 à 18 **caractérisé en ce que** les composés pyridine benzimidazole sont choisis entre un group comprenant Oméprazole, Lansoprazole et Pantoprazole.
